# EUROPÄISCHE PATENTSCHRIFT

(11) **EP 3 691 515 B1**
(45) Veröffentlichungstag und Bekanntmachung des Hinweises auf die Patenterteilung: **02.08.2023**
(21) Anmeldenummer: 18782999.9
(22) Anmeldetag: 05.10.2018
(51) Int. Cl.: A61B 3/06, A61B 3/00, A61B 3/028

(54) **SYSTEM UND VERFAHREN ZUR ERMITTLUNG VON KENNWERTEN EINER AMETROPIE EINES PROBANDEN**
SYSTEM AND METHOD FOR DETERMINING CHARACTERISTIC VALUES OF AMETROPIA OF A TEST PERSON
SYSTÈME ET PROCÉDÉ DE DÉTERMINATION DE VALEURS CARACTÉRISTIQUES D'UNE AMÉTROPIE D'UN SUJET

(30) Priorität: 06.10.2017 DE 102017123301
(43) Veröffentlichungstag der Anmeldung: 12.08.2020
(73) Patentinhaber: Laser Zentrum Hannover e.V., 30419 Hannover (DE)
(72) Erfinder: OBERHEIDE, Uwe, 50674 Köln (DE); WIEL, Gero, 51381 Leverkusen (DE)
(74) Vertreter: Michalski Hüttermann & Partner Patentanwälte mbB
(86) Internationale Anmeldenummer: PCT/EP2018/077219
(87) Internationale Veröffentlichungsnummer: WO 2019/068908

(56) Entgegenhaltungen:
- NEIL MOHON ET AL: "Laser Speckle for Determining Ametropia and Accommodation Response of the Eye", APPLIED OPTICS, Bd. 12, Nr. 4, 1. April 1973 (1973-04-01), Seite 783, XP055537830, WASHINGTON, DC; US ISSN: 0003-6935, DOI: 10.1364/AO.12.000783
- H J Tiziani: "6 Laser-Speckle", Meßtechniken mit Lasern., 1. Januar 1993 (1993-01-01), Seiten 131-159, XP055537732, Ehningen bei Böblingen DOI: 10.18419/opus-4400 ISBN: 978-3-8169-0777-0 Gefunden im Internet: URL:https://elib.uni-stuttgart.de/bitstrea m/11682/4417/1/tiz122.pdf [gefunden am 2018-12-21]
- ERIK INGELSTAM ET AL: "Eye refraction examined by aid of speckle pattern produced by coherent light", VISION RESEARCH., Bd. 12, Nr. 3, 1. März 1972 (1972-03-01), Seiten 411-IX, XP055537825, GB ISSN: 0042-6989, DOI: 10.1016/0042-6989(72)90086-7

## Beschreibung

Die Erfindung betrifft ein System zur Ermittlung von Kennwerten einer Ametropie eines Probanden, mit (i) einer Vorrichtung zum Generieren von optischen Teststrukturen und (ii) einer Einrichtung zur Betrachtung der Teststrukturen durch den Probanden, die eine Korrektureinheit zur Korrektur möglicher Ametropie-Phänomene des Probanden aufweist.

Die Erfindung betrifft weiterhin eine entsprechende Vorrichtung zum Generieren von optischen Teststrukturen zur Ermittlung von Kennwerten einer Ametropie eines Probanden und ein entsprechendes Verfahren zur Ermittlung von Kennwerten einer Ametropie eines Probanden.

Ein derartiges System ist als Phoropter-System bekannt. Der Phoropter ist eine augenoptische Apparatur, mit der die so genannte subjektive Refraktion eines Probanden bestimmt werden kann. Diese wird in der Regel auf der Grundlage objektiv gemessener Fehlsichtigkeiten (Ametropie) ermittelt und zur Anpassung von Brillengläsern oder Kontaktlinsen benötigt. Dabei schaut der Proband von der Rückseite des Geräts durch zwei runde Öffnungen, deren Abstand sich entsprechend dem individuellen Augenabstand anpassen lässt. Der Untersucher nimmt von vorne die notwendigen Einstellungen vor.

Ametropie bezeichnet in der medizinischen Optik den Zustand eines Augapfels, der einen optisch im Unendlichen liegenden Gegenstand bei entspannter Akkommodation nicht scharf auf die Netzhaut abbildet. Dabei sind die folgenden Fälle zu unterscheiden: (a) Als Hyperopie (auch Weitsichtigkeit genannt) wird eine Ametropie bezeichnet, bei der das Abbild eines im optisch Unendlichen liegenden Gegenstandes und parallel einfallenden Lichtstrahlen bei entspannter Akkommodation hinter die Netzhaut zu liegen kommt. (b) Als Myopie (auch Kurzsichtigkeit genannt) wird eine Ametropie bezeichnet, bei der das Abbild des im Unendlichen liegenden Gegenstandes bei entspannter Akkommodation vor die Netzhaut zu liegen kommt. Hyperopie und Myopie werden als sogenannte "Achsen-Ametropien" zusammengefasst. (c) Als Astigmatismus (auch Stabsichtigkeit oder Hornhautverkrümmung genannt) bezeichnet man den Zustand eines Augapfels, bei dem parallel einfallende Lichtstrahlen abhängig von ihrer Einfallsebene unterschiedlich stark gebrochen werden. Dabei stehen die Ebenen mit maximaler und minimaler Brechkraft meist senkrecht aufeinander. Als Stärke des Astigmatismus wird die Brechkraftdifferenz zwischen diesen beiden Ebenen angegeben.

Die Ermittlung von Kennwerten einer Ametropie eines Probanden ist nichts anderes als die Bestimmung der subjektiven Refraktion (oft auch "Abgleich" oder "Brillenabgleich" genannt) des Probanden. Dem Probanden werden nacheinander systematisch verschiedene Linsen vorgehalten und er wird nach einer Verbesserung oder Verschlechterung des Seheindrucks gefragt. Dabei werden in der Regel jene Sehzeichen, die auch für die Bestimmung der Sehschärfe verwendet werden, mittels einer Projektionsvorrichtung als Teststrukturen zur Betrachtung angeboten.

Die Auswahl und das Vorhalten der Linsen lässt sich beschleunigen durch die Verwendung eines Phoropters. Die Anwendung eines Phoropters bietet eine komfortable Möglichkeit, erwachsenen Personen und größeren Kindern verschiedene Glasstärken in Abstufungen von 0.25 Dioptrien anzubieten und so zu prüfen, ob sich die Sehschärfe dadurch verändert. Alle für eine Brillenglasbestimmung notwendigen sphärischen und zylindrischen Glasstärken stehen dabei zur Verfügung. Die Einstellung der Achslage von Zylindergläsern entspricht dem so genannten TABO-Schema von 0° bis 180°. Bedient wird der Phoropter entweder manuell über Drehknöpfe, die an der Vorderseite des Gerätes angebracht sind, oder computergesteuert mittels einer zentralen Steuerkonsole, die mit dem Gerät verbunden ist. Über eine Schnittstelle ist es in diesem Fall auch möglich, im Computer gespeicherte Werte, zum Beispiel frühere Brillenwerte oder die Ergebnisse einer objektiven Refraktionsmessung, direkt an den Phoropter zu senden, der dann diese Werte einstellt.

Zudem ist es mit einem Phoropter möglich, unter Verwendung von Farb- und Polarisationsfiltern Untersuchungen des beidäugigen Sehens hinsichtlich Heterophorie, Simultansehen und Fusion durchzuführen. Zur Korrektur stehen eingebaute Prismen zur Verfügung, sowie Siebtests für spezielle orthoptische Untersuchungen (Worth-Test, Schober-Test).

Der Artikel »N. Mohon et al.:" Laser Speckle for Determining Ametropia and Accommodation Response of the Eye"; APPLIED OPTICS, Bd. 12, Nr. 4, 1. April 1973, Seite 783, WASHINGTON, DC; US« beschreibt ein System zur Ermittlung von Kennwerten einer Ametropie eines Probanden, mit einer Vorrichtung zum Generieren von optischen Teststrukturen und einer Einrichtung zur Betrachtung der Teststrukturen durch den Probanden, die eine Korrektureinheit zur Korrektur möglicher Ametropie-Phänomene des Probanden aufweist, wobei die optischen Teststrukturen mehrere sich bewegende Speckle-Muster aufweisen und diese Speckle-Muster je nach Ausgestaltung der Vorrichtung zum Generieren von optischen Teststrukturen alternative Wellenlängen aufweisen können.

Es ist die Aufgabe der Erfindung Maßnahmen anzugeben, mittels derer eine Ermittlung von Kennwerten einer Ametropie auch für kleinere Kinder und/oder Personen mit geringerer Sehfähigkeit möglich ist.

Die Lösung der Aufgabe erfolgt erfindungsgemäß durch die Merkmale der unabhängigen Ansprüche. Vorteilhafte Ausgestaltungen der Erfindung sind in den Unteransprüchen angegeben.

Bei dem System zur Ermittlung von Kennwerten einer Ametropie eines Probanden, welches eine Vorrichtung zum Generieren von optischen Teststrukturen und eine Einrichtung zur Betrachtung der Teststrukturen durch den Probanden umfasst, die ihrerseits eine Korrektureinheit zur Korrektur möglicher Ametropie-Phänomene des Probanden aufweist, ist erfindungsgemäß vorgesehen, dass die optischen Teststrukturen mindestens zwei sich bewegende Speckle-Muster unterschiedlicher Wellenlänge aufweisen. Sich bewegend heißt in diesem Zusammenhang sich relativ zum Kopf beziehungsweise den Augen des Probanden bewegend. Das System ist ein System zur Ermittlung von Kennwerten der Ametropie des Probanden mittels subjektiver Refraktion.

Bei der Erfindung wird die Bestimmung der Ametropie durch manuelle Bestimmung der Bewegungsrichtung von "Speckle"-Phänomenen (virtuellen Lichtstrukturen) ausgenutzt. Dabei bewegt sich bei einer Relativbewegung Kopf-zu-Speckle-Muster für eine kurzsichtige Person das wahrgenommene Muster entgegengesetzt, bei einer weitsichtigen Person in die gleiche Richtung wie das Muster selbst.

Bei der Erfindung werden nun verschiedene, gleichzeitig dargestellte Speckle-Muster unterschiedlicher Farbe beziehungsweise Wellenlänge genutzt. Dabei wird die chromatische Aberration von Linsen ausgenutzt, die dafür sorgt, dass Licht unterschiedlicher Wellenlänge oder Farbe verschieden stark gebrochen wird. Aus den Bewegungsrichtungen kann über den Vergleich von zwei verschiedenen Wellenlängen, idealerweise rot und grün, die jeweils ein Muster erzeugen, die subjektiv bestwahrgenommene Korrektur bestimmt werden. Bei optimaler Korrektur bewegen sich die beiden Speckle-Muster nicht in die gleiche, sondern in unterschiedliche Richtungen. Hierdurch kann (basierend auf mathematischen Simulationen der Lichtwege) eine Genauigkeit von 0,25 dpt- entsprechend der Genauigkeit von Testbrillen oder Phoroptern erreicht werden.

Bei dem System ist insbesondere vorgesehen, dass die Vorrichtung zum Generieren von optischen Teststrukturen als Projektionsvorrichtung zur Projektion kohärenten Lichts mit mindestens zwei unterschiedlichen Wellenlängen auf eine die Teststrukturen bildende Fläche ausgebildet ist. Die Wellenlängen entsprechen insbesondere den Farben Rot (etwa 650 nm) und Grün (etwa 500 nm).

Die Vorrichtung zum Generieren von optischen Teststrukturen ist insbesondere eingerichtet, die Bewegung der Speckle-Muster durch eine Relativbewegung von der Fläche zu auf der Fläche auftreffenden Lichtstrahlen des Lichts mit den unterschiedlichen Wellenlängen zu generieren.

Gemäß einer vorteilhaften Ausgestaltung der Erfindung weist die Korrektureinheit mindestens einen Satz an unterschiedlichen Korrekturlinsen zur Korrektur möglicher Ametropie-Phänomene des Probanden auf. Die Einrichtung zur Betrachtung der Teststrukturen durch den Probanden ist insbesondere eine sogenannte Testbrille oder ein Phoropter.

Bei der erfindungsgemäßen Vorrichtung zum Generieren von optischen Teststrukturen für die Ermittlung von Kennwerten einer Ametropie eines Probanden ist vorgesehen, dass die Teststrukturen mindestens zwei sich bewegende Speckle-Muster unterschiedlicher Wellenlänge aufweisen. Mit anderen Worten ist die Vorrichtung eine Vorrichtung zum Generieren von mindestens zwei sich bewegenden Speckle-Mustern unterschiedlicher Wellenlänge umfassenden optischen Teststrukturen. Die Vorrichtung ist bevorzugt eine Vorrichtung für ein vorgenanntes System zur Ermittlung von Kennwerten einer Ametropie eines Probanden. Die Ermittlung von Kennwerten der Ametropie eines Probanden ist eine Ermittlung mittels subjektiver Refraktion.

Gemäß einer vorteilhaften Ausgestaltung der Erfindung ist vorgesehen, dass die Vorrichtung als Projektionsvorrichtung zur Projektion kohärenten Lichts mit mindestens zwei unterschiedlichen Wellenlängen auf eine die Teststrukturen bildende (Bild- beziehungsweise Projektions-)Fläche ausgebildet ist.

Gemäß einer weiteren vorteilhaften Ausgestaltung der Erfindung ist vorgesehen, dass die Vorrichtung eingerichtet ist, die Bewegung der Speckle-Muster durch eine Relativbewegung von der Fläche zu auf der Fläche auftreffenden Lichtstrahlen des Lichts mit den unterschiedlichen Wellenlängen zu generieren. Dazu weist die Fläche eine hinreichende Oberflächenrauhigkeit oder andere Struktur auf.

Dabei ist insbesondere vorgesehen, dass die Vorrichtung zumindest eine Ablenkeinheit zum Bewegen der Lichtstrahlen des Lichts mit den unterschiedlichen Wellenlängen aufweist. Diese Ablenkeinheit ist bevorzugt in Form eines Polygonscanners oder Galvoscanners ausgestaltet.

Gemäß einer vorteilhaften Ausführungsform der erfindungsgemäßen Vorrichtung ist vorgesehen, dass die Vorrichtung ein die Fläche bereitstellendes Element, insbesondere ein eine holographische Struktur aufweisendes Element, aufweist.

Gemäß einer weiteren vorteilhaften Ausführungsform der erfindungsgemäßen Vorrichtung weist diese Vorrichtung einen Apparat zum Bewegen des die Fläche bereitstellenden Elements auf.

Bei dem erfindungsgemäßen Verfahren zur Ermittlung von Kennwerten einer Ametropie eines Probanden sind die folgenden Schritte vorgesehen: (i) ein Generieren von optischen Teststrukturen, wobei die optischen Teststrukturen mindestens zwei sich bewegende Speckle-Muster unterschiedlicher Wellenlänge aufweisen und (ii) eine Betrachtung der Teststrukturen durch den Probanden, wobei eine Korrektur der Ametropie-Phänomene des Probanden mittels einer Korrektureinheit zur Korrektur möglicher Ametropie-Phänomene durchgeführt wird. Das Verfahren ist ein Verfahren zur Ermittlung von Kennwerten der Ametropie des Probanden mittels subjektiver Refraktion.

Gemäß einer vorteilhaften Ausgestaltung der Erfindung ist vorgesehen, dass das Verfahren mittels des vorstehend genannten Systems durchgeführt wird. Dies ist mit anderen Worten die Verwendung des vorstehend genannten Systems zur Ermittlung von Kennwerten einer Ametropie (der Augen) eines Probanden.

Nachfolgend wird die Erfindung unter Bezugnahme auf die anliegenden Zeichnungen anhand von bevorzugten Ausführungsbeispielen exemplarisch erläutert, wobei die nachfolgend dargestellten Merkmale sowohl jeweils einzeln als auch in Kombination einen Aspekt der Erfindung darstellen können. Es zeigen:
Fig. 1 eine schematische Darstellung eines Speckle-Muster betrachtenden weitsichtigen Auges,
Fig. 2 eine schematische Darstellung eines Speckle-Muster betrachtenden emmetropen Auges,
Fig. 3 ein System zur Ermittlung von Kennwerten einer Ametropie eines Probanden gemäß einer bevorzugten Ausführungsform der Erfindung,
Fig. 4 eine erste Ausgestaltung einer Ablenkeinheit einer Vorrichtung zum Generieren sich bewegender Speckle-Muster,
Fig. 5 eine zweite Ausgestaltung einer Ablenkeinheit einer Vorrichtung zum Generieren sich bewegender Speckle-Muster,
Fig. 6 eine Ausgestaltung eines Apparats zum Bewegen der die Speckle-Muster bildenden Bildwand und
Fig. 7 eine weitere Vorrichtung zum Generieren sich bewegender Speckle-Muster.

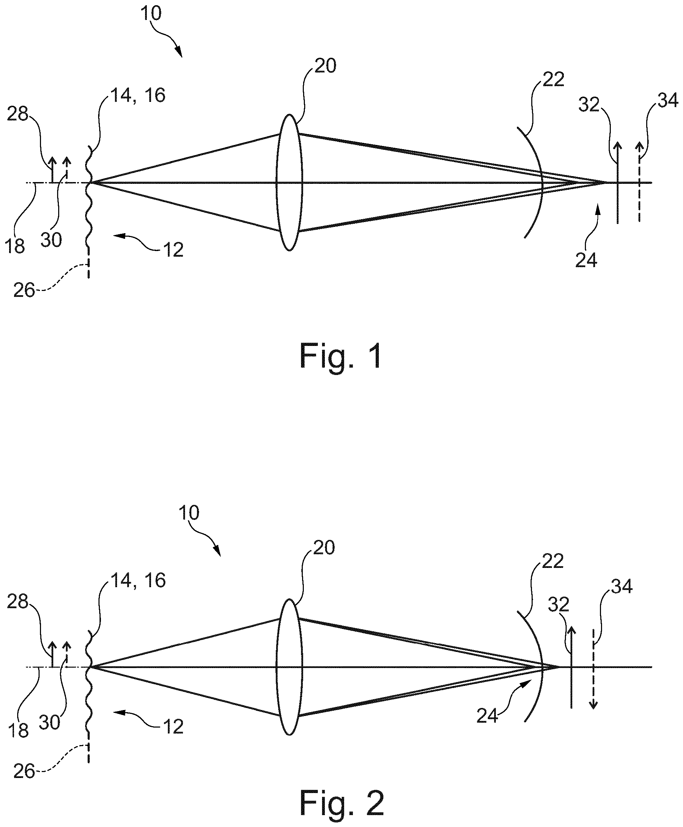

Die Fig. 1 zeigt in schematischer Darstellung ein weitsichtiges Auge 10 eines Probanden, der sich optische Teststrukturen 12 mit zwei jeweils bezüglich des Auges 10 beziehungsweise des Kopfes des Probanden bewegenden Speckle-Mustern 14, 16 betrachtet. Das Auge 10 ist dabei über seine optische Achse 18, seine Linse 20 sowie seine Netzhaut (Retina) 22 definiert. Die Hornhaut des Auges 10 und andere Details sind in dieser stark vereinfachenden Darstellung weggelassen. Da es sich um ein weitsichtiges Auge 10 handelt, liegt dessen Fokus 24 hinter der Netzhaut 22. Die beiden Speckle-Muster 14, 16 bewegen sich in der gleichen Gegenstandsebene 26, weisen jedoch unterschiedliche Farben beziehungsweise Wellenlängen auf. Die Bewegung des Speckle-Musters 14 höherer Wellenlänge (zum Beispiel 650 nm - Rot) ist über den Pfeil 28 angedeutet, die Bewegung des Speckle-Musters 16 niedrigerer Wellenlänge (zum Beispiel 500 nm - Grün) über den Pfeil 30. Im gezeigten Beispiel bewegen sich die Speckle-Muster 14, 16 mit gleicher Geschwindigkeit in die gleiche Richtung. Die vom Probanden wahrgenommene Bewegung der Speckle-Muster 14, 16 wird durch die Pfeile 32, 34 repräsentiert. Dabei gibt Pfeil 32 die wahrgenommene Bewegung des Speckle-Musters 14 höherer Wellenlänge und Pfeil 34 die wahrgenommene Bewegung des Speckle-Musters 16 niedrigerer Wellenlänge an. Der weitsichtige Proband nimmt die Bewegung der Speckle-Muster 14, 16 deutlich wahr, wobei die wahrgenommene Bewegungsrichtung der tatsächlichen Bewegungsrichtung der Speckle-Muster 14, 16 entspricht. Dies liegt daran, dass sich der Fokus 24 hinter der Netzhaut 22 befindet.

Anders sieht die Situation beim kurzsichtigen Auge 10 aus (nicht gezeigt). Hier liegt der Fokus 24 vor der Netzhaut 22. Der kurzsichtige Proband nimmt die Bewegung der Speckle-Muster 14, 16 ebenfalls deutlich wahr, wobei die wahrgenommene Bewegungsrichtung gegenüber der tatsächlichen Bewegungsrichtung der Speckle-Muster 14, 16 jedoch umgekehrt ist.

Die Fig. 2 zeigt nun die der Fig. 1 entsprechende Situation für das emmetrope Auge 10 beziehungsweise für das weitsichtige Auge 10, bei dem die Hyperopie durch eine zusätzliche Linse, zum Beispiel eine Kontaktlinse oder Brille, vollkommen ausgeglichen ist. Bei Fehlen einer Ametropie oder einem vollständigen Ausgleich der Ametropie kommt nun die chromatische Aberration des optischen Systems (zum Beispiel der Linse 20) zum Tragen. Ist die Ametropie für eine mittlere Wellenlänge (zwischen der höheren und der niedrigeren Wellenlänge) vollständig korrigiert, so entspricht die wahrgenommene Bewegungsrichtung des Speckle-Musters 14 höherer Wellenlänge weiterhin der tatsächlichen Bewegungsrichtung dieses Speckle-Musters 14, wohingegen die wahrgenommene Bewegungsrichtung des Speckle-Musters 16 niedrigerer Wellenlänge gegenüber der tatsächlichen Bewegungsrichtung dieses Speckle-Musters 16 umgekehrt ist. Der Proband hat in dieser Situation den Eindruck, dass sich beide Speckle-Muster 14, 16 gegenläufig bewegen oder dass beide Speckle-Muster 14, 16 fix sind.

Dieses Phänomen kann nun mit Hilfe eines im Folgenden beschriebenen Systems 36 zur Ermittlung von Kennwerten der Ametropie des Probanden genutzt werden.

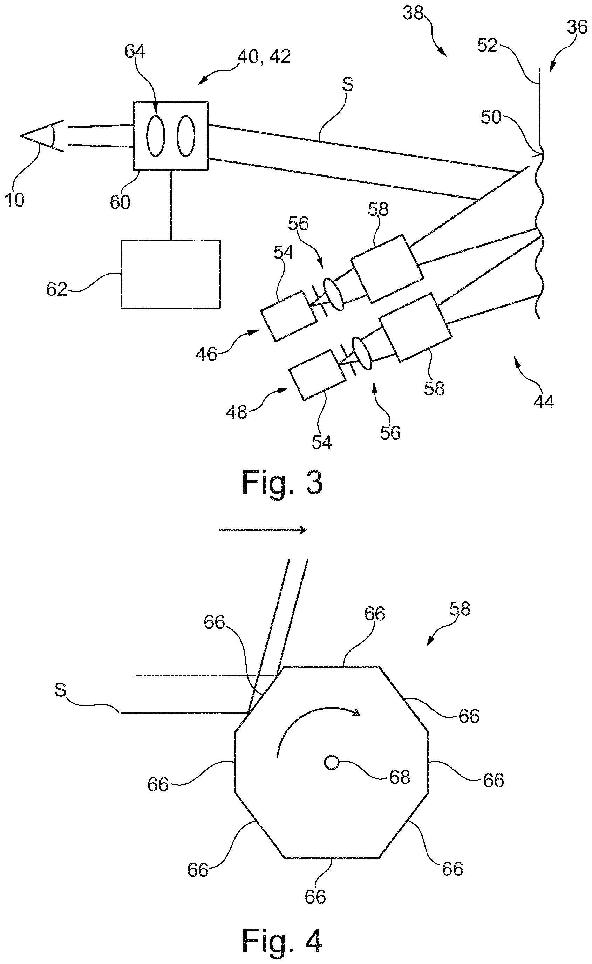

Die Fig. 3 zeigt ein Beispiel für ein solches System 36 zur Ermittlung von Kennwerten der Ametropie eines Probanden. Das System umfasst (i) eine Vorrichtung 38 zum Generieren der optischen Teststrukturen 12 mit den zwei sich bewegende Speckle-Mustern 14, 16 unterschiedlicher Wellenlänge und (ii) eine als Phoropter 40 ausgestaltete Einrichtung 42 zur Betrachtung dieser Teststrukturen 12 durch den Probanden.

Die Vorrichtung 38 zum Generieren der optischen Teststrukturen 12 mit den zwei sich bewegende Speckle-Mustern 14, 16 unterschiedlicher Wellenlänge weist eine Projektionsvorrichtung 44 mit zwei Projektionsmodulen 46, 48 zur Projektion kohärenten Lichts mit unterschiedlichen Wellenlängen sowie eine die Teststrukturen bildende Bildwandfläche 50 auf. Diese Fläche 50 wird beispielsweise von einer Bildwand (auch Projektionswand genannt) 52 mit entsprechender Oberflächenstruktur gebildet. Die Bildwandfläche 50 beziehungsweise Bildwand 52 kann als separate Systemkomponente des Systems 36 als auch als Teil der Vorrichtung 38 angesehen werden. Jedes der Projektionsmodule 46, 48 weist eine Lichtquelle 54 für kohärentes Licht entsprechender Wellenlänge, je eine der Lichtquelle 54 nachgeschaltete Optik 56 sowie je eine Ablenkeinheit 58 zum Bewegen der Lichtstrahlen des Lichts entsprechender Wellenlänge auf. Es ergibt sich der Strahlengang S.

Die Einrichtung 42 zur Betrachtung von Teststrukturen weist eine Korrektureinheit 60 zur Korrektur möglicher Ametropie-Phänomene des Probanden sowie ein Betätigungselement 62 zum Betätigen der Korrektureinheit 60 auf. Die Korrektureinheit 60 umfasst einen Satz an unterschiedlichen Korrekturlinsen 64 für die Korrektur.

Zur Ermittlung von Kennwerten der Ametropie der Augen 10 eines Probanden werden (i) mittels der Vorrichtung 38 optische Teststrukturen 12 generiert, wobei die optischen Teststrukturen 12 zwei sich bewegende Speckle-Muster 14, 16 unterschiedlicher Wellenlänge aufweisen, (ii) diese Teststrukturen 12 vom Probanden über die Betrachtung-Einrichtung 42 mit der Korrektureinheit 60 betrachtet und (iii) Korrekturen der Ametropie-Phänomene des Probanden mittels der Korrektureinheit 60 durchgeführt.

Die Fig. 4 zeigt eine erste Ausgestaltung der Ablenkeinheit 58 der Vorrichtung 38 zum Generieren sich bewegender Speckle-Muster 14, 16. Bei dieser Ausgestaltung ist die Ablenkeinheit 58 als ein Spiegelsystem in der Art eines Polygonscanners ausgebildet. Im gezeigten Beispiel sind acht Spiegelelemente 66 in Form eines Achtecks angeordnet, welches um die Drehachse 68 drehbar gelagert ist beziehungsweise gedreht wird (Pfeil). Auf diese Weise kann ein oder mehrere Lichtstrahlen bewegt werden.

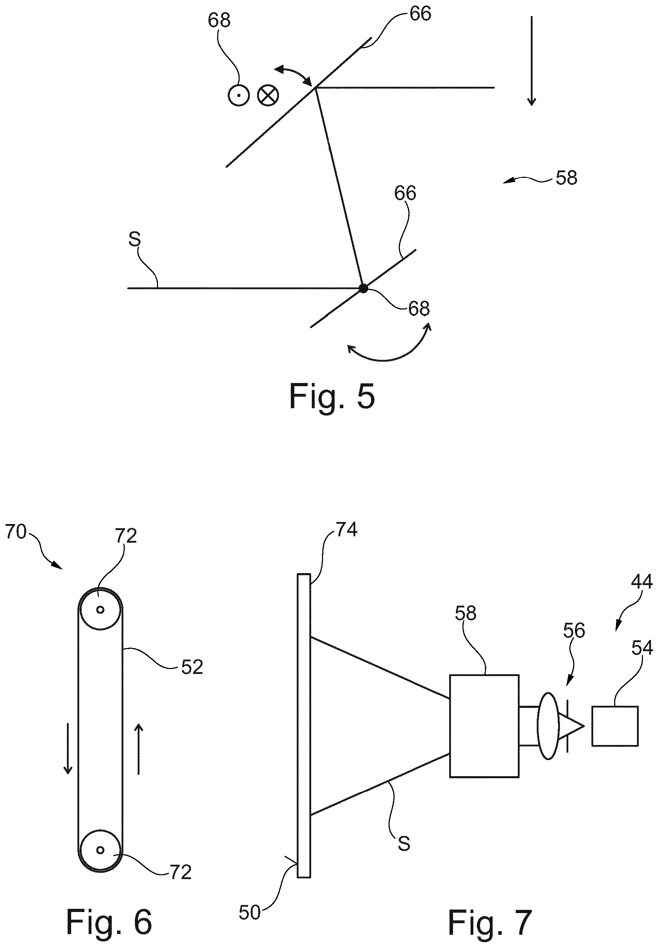

Die Fig. 5 zeigt eine zweite Ausgestaltung der Ablenkeinheit 58 der Vorrichtung zum Generieren sich bewegender Speckle-Muster 14, 16. Bei dieser Ausgestaltung ist die Ablenkeinheit 58 als ein Spiegelsystem in der Art eines Galvo-Scanners ausgebildet. Im gezeigten Beispiel sind zwei Spiegelelemente 66 (x- und y-Spiegelelement) um entsprechende Drehachsen 68 drehbar gelagert. Auf diese Weise können ein oder mehrere Lichtstrahlen bewegt werden.

Die Fig. 6 zeigt eine Ausgestaltung eines Apparats 70 zum Bewegen der die Speckle-Muster 14, 16 bildenden Bildwand 52. Diese ist als Bildwand in Form eines Umschlingungsmittels ausgebildet, welches zwei Rollen 72 umschlingt. Zumindest eine der Rollen 72 ist als Antriebsrolle ausgebildet und lässt die Bildwand über die Rollen 72 abrollen (Pfeil).

Die Fig. 7 zeigt schließlich eine weitere Vorrichtung 38 zum Generieren sich bewegender Speckle-Muster 14, 16. Diese weist eine Projektionsvorrichtung 44 mit zwei Lichtquellen 54 für kohärentes Licht entsprechender Wellenlängen, den Lichtquellen 54 nachgeschaltete Optiken 56 und eine Ablenkeinheit 58 zum Bewegen der Lichtstrahlen des Lichts sowie ein die Bildwandfläche 50 bereitstellendes Element 74, welches eine holographische Struktur aufweist, also ein Hologramm. Die Verwendung eines eine holographische Struktur aufweisenden Elements 74 zum Bereitstellen der Bildwandfläche 50 ermöglicht es, dass -wie hier gezeigt- von hinten, also von der der Fläche 50 abgewandten Seite des Elements 74 von der Projektionsvorrichtung 44 beleuchtet werden kann.

### Bezugszeichen

- 10: Auge
- 12: optische Teststruktur
- 14: Speckle-Muster
- 16: Speckle-Muster
- 18: optische Achse (Auge)
- 20: Linse (Auge)
- 22: Netzhaut
- 24: Fokus
- 26: Gegenstandsebene
- 28: Pfeil (Bewegung Speckle-Muster höherer Wellenlänge)
- 30: Pfeil (Bewegung Speckle-Muster niedrigerer Wellenlänge)
- 32: Pfeil (wahrgenommene Bewegung Speckle-Muster höherer Wellenlänge)
- 34: Pfeil (wahrgenommene Bewegung Speckle-Muster niedrigerer Wellenlänge)
- 36: System zur Ermittlung von Kennwerten einer Ametropie
- 38: Vorrichtung zum Generieren optischer Teststrukturen
- 40: Phoropter
- 42: Einrichtung zur Betrachtung von Teststrukturen
- 44: Projektionsvorrichtung
- 46: Projektionsmodul
- 48: Projektionsmodul
- 50: Bildwandfläche
- 52: Bildwand
- 54: Lichtquelle
- 56: Optik
- 58: Ablenkeinheit
- 60: Korrektureinheit
- 62: Betätigungselement
- 64: Satz an Korrekturlinsen
- 66: Spiegel
- 68: Drehachse
- 70: Apparat zum Bewegen der Bildwand
- 72: Rollen
- 74: Hologramm
- S: Strahlengang

## Patentansprüche

1. System (36) zur Ermittlung von Kennwerten einer Ametropie eines Probanden, mit
- einer Vorrichtung (38) zum Generieren von optischen Teststrukturen (12) und
- einer Einrichtung (42) zur Betrachtung der Teststrukturen (12) durch den Probanden, die eine Korrektureinheit (60) zur Korrektur möglicher Ametropie-Phänomene des Probanden aufweist,
**dadurch gekennzeichnet, dass** die optischen Teststrukturen (12) mindestens zwei verschiedene, gleichzeitig dargestellte und sich bewegende Speckle-Muster (14, 16) unterschiedlicher Wellenlänge aufweisen.

2. System nach Anspruch 1, **dadurch gekennzeichnet, dass** die Vorrichtung zum Generieren von optischen Teststrukturen (12) als Projektionsvorrichtung (44) zur Projektion kohärenten Lichts mit mindestens zwei unterschiedlichen Wellenlängen auf eine die Teststrukturen bildende Bildwandfläche (50) ausgebildet ist.

3. System nach Anspruch 1 oder 2, **dadurch gekennzeichnet, dass** die Korrektureinheit (60) mindestens einen Satz an unterschiedlichen Korrekturlinsen (64) zur Korrektur möglicher Ametropie-Phänomene des Probanden aufweist.

4. Vorrichtung (38) zum Generieren von optischen Teststrukturen (12) für die Ermittlung von Kennwerten einer Ametropie eines Probanden, insbesondere für ein System (36) nach einem der Ansprüche 1 bis 3, **dadurch gekennzeichnet, dass** die Teststrukturen (12) mindestens zwei verschiedene, gleichzeitig dargestellte und sich bewegende Speckle-Muster (14, 16) unterschiedlicher Wellenlänge aufweisen.

5. Vorrichtung nach Anspruch 4, **dadurch gekennzeichnet, dass** die Vorrichtung (38) als Projektionsvorrichtung (44) zur Projektion kohärenten Lichts mit mindestens zwei unterschiedlichen Wellenlängen auf eine die Teststrukturen (12) bildende Bildwandfläche (50) ausgebildet ist.

6. Vorrichtung nach Anspruch 5, **dadurch gekennzeichnet, dass** die Vorrichtung (38) eingerichtet ist, die Bewegung der Speckle-Muster (14, 16) durch eine Relativbewegung von der Bildwandfläche (50) zu auf dieser Fläche (50) auftreffenden Lichtstrahlen des Lichts mit den unterschiedlichen Wellenlängen zu generieren.

7. Vorrichtung nach Anspruch 6, **gekennzeichnet durch** zumindest eine Ablenkeinheit (58) zum Bewegen der Lichtstrahlen des Lichts mit den unterschiedlichen Wellenlängen.

8. Vorrichtung nach einem der Ansprüche 5 bis 7, **gekennzeichnet durch** ein die Bildwandfläche (50) bereitstellendes Element (52, 74), insbesondere ein eine holographische Struktur aufweisendes Element (74).

9. Vorrichtung nach Anspruch 8, **gekennzeichnet durch** einen Apparat (70) zum Bewegen des die Bildwandfläche (50) bereitstellenden Elements (52).

10. Verfahren zur Ermittlung von Kennwerten einer Ametropie eines Probanden, durch
- ein Generieren von optischen Teststrukturen (12), wobei die optischen Teststrukturen (12) mindestens zwei verschiedene, gleichzeitig dargestellte und sich bewegende Speckle-Muster (14, 16) unterschiedlicher Wellenlänge aufweisen
- eine Betrachtung der Teststrukturen (12) durch den Probanden und
eine Korrektur der Ametropie-Phänomene des Probanden mittels einer Korrektureinheit (60) zur Korrektur möglicher Ametropie-Phänomene.

## Claims

1. System (36) for determining characteristic values of an ametropia of a subject, having
- a device (38) for generating optical test structures (12) and
- a means (42) for viewing the test structures (12) by the subject, comprising a correction unit (60) for correcting possible ametropia phenomena of the subj ect,
**characterized in that** the optical test structures (12) have at least two different simultaneously displayed and moving speckle patterns (14, 16) of different wavelengths.

2. System according to claim 1, **characterized in that** the device for generating optical test structures (12) is designed as a projection device (44) for projecting coherent light with at least two different wavelengths onto an image wall surface (50) forming the test structures.

3. System according to claim 1 or 2, **characterized in that** the correction unit (60) comprises at least one set of different correction lenses (64) for correcting possible ametropia phenomena of the subject.

4. Device (38) for generating optical test structures (12) for determining characteristic values of an ametropia of a subject, in particular for a system (36) according to one of claims 1 to 3, **characterized in that** the test structures (12) have at least two different, simultaneously displayed and moving speckle patterns (14, 16) of different wavelengths.

5. Device according to claim 4, **characterized in that** the device (38) is designed as a projection device (44) for projecting coherent light with at least two different wavelengths onto an image wall surface (50) forming the test structures (12).

6. Apparatus according to claim 5, **characterized in that** the apparatus (38) is arranged to generate the movement of the speckle patterns (14, 16) by a relative movement from the image wall surface (50) to light rays of the light with the different wavelengths impinging on this surface (50).

7. Apparatus according to claim 6, **characterized by** at least one deflection unit (58) for moving the light beams of the light having the different wavelengths.

8. Device according to any one of claims 5 to 7, **characterized by** an element (52, 74) providing the image wall surface (50), in particular an element (74) having a holographic structure.

9. Apparatus according to claim 8, **characterized by** an apparatus (70) for moving the member (52) providing the image wall surface (50).

10. Method for determining characteristic values of an ametropia of a subject, by means of
- generating optical test structures (12), the optical test structures (12) having at least two simultaneously displayed and moving speckle patterns (14, 16) of different wavelengths
- observation of the test structures (12) by the subject's and
a correction of the ametropia phenomena of the subject by means of a correction unit (60) for correcting possible ametropia phenomena.

## Revendications

1. Système (36) de détermination de valeurs caractéristiques d'une amétropie d'un sujet, pourvu
- d'un dispositif (38) de génération de structures de test (12) optiques et
- d'un équipement (42) de visualisation des structures de test (12) par le sujet, qui présente une unité de correction (60) permettant la correction de phénomènes d'amétropie éventuels du sujet,
**caractérisé en ce que** les structures de test (12) optiques présentent au moins deux motifs de tavelure (14, 16) différents, représentés simultanément et mobiles, de longueurs d'onde différentes.

2. Système selon la revendication 1, **caractérisé en ce que** le dispositif de génération de structures de test (12) optiques est conçu sous forme de dispositif de projection (44) permettant la projection de lumière cohérente avec au moins deux longueurs d'onde différentes sur une surface d'écran (50) formant les structures de test.

3. Système selon la revendication 1 ou la revendication 2, **caractérisé en ce que** l'unité de correction (60) présente au moins un ensemble de lentilles de correction (64) différentes pour la correction de phénomènes d'amétropie éventuels du sujet.

4. Dispositif (38) de génération de structures de test (12) optiques permettant la détermination de valeurs caractéristiques d'une amétropie d'un sujet, en particulier pour un système (36) selon l'une des revendications 1 à 3, **caractérisé en ce que** les structures de test (12) présentent au moins deux motifs de tavelure (14, 16) différents, représentés simultanément et mobiles, de longueurs d'onde différentes.

5. Dispositif selon la revendication 4, **caractérisé en ce que** le dispositif (38) est conçu sous forme de dispositif de projection (44) permettant la projection de lumière cohérente avec au moins deux longueurs d'onde différentes sur une surface d'écran (50) formant les structures de test (12).

6. Dispositif selon la revendication 5, **caractérisé en ce que** le dispositif (38) est conçu pour générer le déplacement des motifs de tavelures (14, 16) par un déplacement relatif de la surface d'écran (50) vers des faisceaux lumineux de la lumière pourvus des longueurs d'onde différentes tombant sur cette surface (50).

7. Dispositif selon la revendication 6, **caractérisé par** au moins une unité de diffraction (58) permettant de déplacer les faisceaux lumineux de la lumière avec des longueurs d'onde différentes.

8. Dispositif selon l'une des revendications 5 à 7, **caractérisé par** un élément (52, 74) fournissant la surface d'écran (50), en particulier, un élément (74) présentant une structure holographique.

9. Dispositif selon la revendication 8, **caractérisé par** un appareil (70) permettant le déplacement de l'élément (52) fournissant la surface d'écran (50).

10. Procédé de détermination de valeurs caractéristiques d'une amétropie d'un sujet par
- une génération de structures de test (12) optiques, où les structures de test (12) optiques présentent au moins deux motifs de tavelure (14, 16) différents, représentés simultanément et mobiles, de longueurs d'onde différentes,
- une visualisation des structures de test (12) par le sujet, et
une correction des phénomènes d'amétropie du sujet au moyen d'une unité de correction (60) permettant la correction de phénomènes d'amétropie éventuels.
